# EUROPÄISCHE PATENTSCHRIFT

(11) **EP 2 996 710 B1**
(45) Veröffentlichungstag und Bekanntmachung des Hinweises auf die Patenterteilung: **03.03.2021**
(21) Anmeldenummer: 14719793.3
(22) Anmeldetag: 29.04.2014
(51) Int. Cl.: A61K 38/39, A61K 38/01, A61P 21/00

(54) **WIRKSTOFF ZUR BEHANDLUNG VON SARKOPENIE**
ACTIVE SUBSTANCE FOR TREATING SARCOPENIA
SUBSTANCE ACTIVE POUR LE TRAITEMENT DE LA SARCOPÉNIE

(30) Priorität: 13.05.2013 DE 102013104897
(43) Veröffentlichungstag der Anmeldung: 23.03.2016
(73) Patentinhaber: Gelita AG, 69412 Eberbach (DE)
(72) Erfinder: HAUSMANNS, Stephan, 69126 Heidelberg (DE); KNEFÉLI, Hans-Christoph, 69257 Wiesenbach (DE); OESSER, Steffen, 24960 Glücksburg (DE); FRECH, Hans-Ulrich, 69469 Weinheim (DE)
(74) Vertreter: Hoeger, Stellrecht & Partner Patentanwälte mbB
(86) Internationale Anmeldenummer: PCT/EP2014/058673
(87) Internationale Veröffentlichungsnummer: WO 2014/183989

(56) Entgegenhaltungen:
- DE-A1-102010 060 564
- DE-A1-102012 110 612
- BELLO A E ET AL: "Collagen hydrolysate for the treatment of osteoarthritis and other joint disorders: A review of the literature", CURRENT MEDICAL RESEARCH AND OPINION, INFORMA HEALTHCARE, GB, Bd. 22, Nr. 11, 1. November 2006 (2006-11-01), Seiten 2221-2232, XP009132739, ISSN: 0300-7995, DOI: 10.1185/030079906X148373 [gefunden am 2006-10-10]
- THE SOCIETY FOR SARCOPENIA CACHEXIA AND WASTING DISEASE ET AL: "Nutritional Recommendations for the Management of Sarcopenia", JOURNAL OF THE AMERICAN MEDICAL DIRECTORS ASSOCIATION, ELSEVIER, NL, Bd. 11, Nr. 6, 1. Juli 2010 (2010-07-01), Seiten 391-396, XP027137147, ISSN: 1525-8610, DOI: 10.1016/J.JAMDA.2010.04.014 [gefunden am 2010-07-11]
- HAYS N P ET AL: "Effects of Whey and Fortified Collagen Hydrolysate Protein Supplements on Nitrogen Balance and Body Composition in Older Women", JOURNAL OF THE AMERICAN DIETETIC ASSOCIATION, THE ASSOCIATION, CHICAGO, IL, US, Bd. 109, Nr. 6, 1. Juni 2009 (2009-06-01), Seiten 1082-1087, XP026132574, ISSN: 0002-8223, DOI: 10.1016/J.JADA.2009.03.003 [gefunden am 2009-05-21]
- Sam Eu: "Peptan B 2000 LD Bovine Collagen Peptides", , 1 October 2012 (2012-10-01), pages 1-2, XP055510952, Retrieved from the Internet: URL:http://www.parmentier.de/gpfneu/gelati ne/B_LD.pdf [retrieved on 2018-09-28]
- DELMONICO MATTHEW J ET AL: "Longitudinal study of muscle strength, quality, and adipose tissue infiltration.", THE AMERICAN JOURNAL OF CLINICAL NUTRITION DEC 2009, vol. 90, no. 6, December 2009 (2009-12), pages 1579-1585, ISSN: 1938-3207
- LIM SOO ET AL: "Association between body composition and pulmonary function in elderly people: the Korean Longitudinal Study on Health and Aging.", OBESITY (SILVER SPRING, MD.) MAR 2011, vol. 19, no. 3, March 2011 (2011-03), pages 631-638, ISSN: 1930-739X
- DENISE ZDZIEBLIK ET AL: "Collagen peptide supplementation in combination with resistance training improves body composition and increases muscle strength in elderly sarcopenic men: a randomised controlled trial", BRITISH JOURNAL OF NUTRITION, vol. 114, no. 8, 28 October 2015 (2015-10-28), pages 1237-1245, XP055414225, UK ISSN: 0007-1145, DOI: 10.1017/S0007114515002810

## Beschreibung

Die vorliegende Erfindung betrifft einen Wirkstoff zur Behandlung von Sarkopenie.

Mit dem Begriff Sarkopenie bezeichnet man einen fortschreitenden Verlust von Muskelmasse und eine Abnahme der Muskelkraft beim Menschen insbesondere mit zunehmendem Alter, wobei das Phänomen regelmäßig ab einem Alter von ca. 50 Jahren oder mehr zu beobachten ist (siehe z.B. Walston, Curr. Opin. Rheumatol. 2012(24)623-627). Als primäre Ursache für die Sarkopenie wird nach derzeitigem Stand ein altersbedingtes Ungleichgewicht zwischen den muskelaufbauenden (anabolen) und den muskelabbauenden (katabolen) Stoffwechselprozessen angenommen. Daneben wird der Muskelabbau offensichtlich durch mangelnde Bewegung und/oder Mangelernährung, die mit höherem Alter ebenfalls häufiger auftreten, begünstigt.

Um der Sarkopenie entgegenzuwirken, wird daher zum einen die Stimulation des Muskelaufbaus durch körperliche Betätigung empfohlen, insbesondere ein gezieltes Muskeltraining bzw. Krafttraining, z.B. an entsprechenden Fitnessgeräten im Rahmen einer medizinischen Trainingstherapie. Ein weiterer Ansatzpunkt, um die muskelaufbauenden Stoffwechselprozesse zu unterstützen, besteht in einer proteinreichen Diät bzw. einer Nahrungsergänzung durch bestimmte Proteine oder deren Hydrolysate, wobei es Hinweise gibt, dass insbesondere die verzweigtkettigen Aminosäuren Leucin, Isoleucin und Valin eine besondere Rolle spielen (siehe z.B. Leenders et al., Nutr. Rev. 2011(69)675-689). Molkenprotein oder Sojaprotein, die einen relativ hohen Anteil dieser Aminosäuren enthalten, werden daher häufig als Nahrungsergänzung eingesetzt. Die Verabreichung isolierter Aminosäuren ist ebenfalls möglich, aber mit deutlich höheren Kosten verbunden.

Die DE 10 2010 060 564 A1 offenbart die Verwendung von Kollagenhydrolysat zur Verbesserung der Gesundheit der menschlichen Haut, Haare und/oder Nägel, wobei mindestens 70 Gew.% des Kollagenhydrolysats ein Molekulargewicht von weniger als ca. 3.500 Da aufweisen.

Morley et al. beschreiben, dass eine adäquate Proteinzufuhr (mit Leucin angereicherte, ausgewogene Aminosäuren und gegebenenfalls Kreatin) die Muskelkraft verbessern können ("Nutritional Recommendations for the Management of Sarcopenia", J. Am. Med. Dir. Assoc. 2010 (11) 391-396).

Hays et al. beschreiben, dass ein konzentriertes, angereichertes, hydrolisiertes Kollagen als Proteinergänzung während 15 Tagen einer relativ proteinarmen Diät das Stickstoffgleichgewicht aufrechterhielt ("Effects of whey and Fortified Collagen Hydrolysate Protein Supplements on Nitrogen Balance and Body Composition in Older Women", J. Am. Diet. Assoc. 2009 (109) 1082-1087).

Die WO 2012/143324 A1 offenbart ein Verfahren zur Herstellung eines Knorpelproduktes, umfassend ein Proteinhydrolysat mit einem Hydrolysegrad zwischen 0,5% und 3,0%, mindestens ein Glykosaminoglykan und mindestens einen Wachstumfaktor. Offenbart wird auch die Verwendung des Knorpelprodukts bei der Behandlung oder Vorbeugung von Wunden, Geschwüren, Brandwunden, Psoriasis, Osteoarthritis, Synovitis, Osteoporose, Osteopenie, Knochenbrüchen, Krankheiten oder Verletzungen von Sehnen oder Ligamenten, periodontalen Krankheiten, vorzugsweise Periodontitis, Muskelerkrankungen, vorzugsweise Muskelathrophie, oder der schädlichen Wirkung von Ultraviolettstrah lenexposition.

Die Erfinder haben nun überraschenderweise festgestellt, dass auch Kollagenhydrolysat bei der Behandlung von Sarkopenie wirksam ist, und zwar bereits in relativ geringen Mengen bezogen auf die empfohlene tägliche Proteinaufnahme.

Ein wesentlicher Aspekt der vorliegenden Erfindung betrifft somit Kollagenhydrolysat als Wirkstoff zur Behandlung von Sarkopenie oder von degenerativem Verlust von Muskelmasse gemäß Anspruch 1.

Ein weiterer Aspekt betrifft die nicht-therapeutische Verwendung von Kollagenhydrolysat als Wirkstoff gemäß den Ansprüchen 2, 3 und 4.

Kollagenhydrolysat, welches insbesondere durch die enzymatische Hydrolyse von kollagenhaltigen tierischen Ausgangsmaterialien hergestellt wird, besteht aus einem Gemisch von Peptiden, deren Molekulargewichte je nach Ausgangsmaterial und Herstellungsbedingungen über einen bestimmten Größenbereich verteilt sind. Die Verwendung von Kollagenhydrolysat als Nahrungsergänzungsmittel ist seit längerem bekannt, und zwar zur Vorbeugung und/oder Behandlung von Beschwerden, die im Zusammenhang mit dem Knochen, den Gelenken oder dem Bindegewebe stehen, zumal eine stimulierende Wirkung der Kollagenpeptide auf die Synthese der körpereigenen extrazellulären Matrix in diesen Gewebetypen gezeigt werden konnte (siehe z.B. Bello et al., Curr. Med. Res. Opin. 2006(22)2221-2232). Unter ernährungsphysiologischen Gesichtspunkten gilt Kollagenhydrolysat allerdings als minderwertige Proteinquelle, da seine Aminosäurezusammensetzung sehr unausgewogen ist und es fast alle essentiellen Aminosäuren nur in sehr geringen Mengen enthält, so auch die verzweigtkettigen Aminosäuren Leucin, Isoleucin und Valin. Das essentielle Tryptophan ist im Kollagenhydrolysat überhaupt nicht enthalten.

Die vorteilhafte Wirkung von Kollagenhydrolysat bei der Behandlung von Sarkopenie, insbesondere beim Aufbau von Muskelmasse und bei der Verbesserung der Muskelkraft, die durch eine klinische Studie belegt werden konnte (siehe unten), war vor diesem Hintergrund nicht zu erwarten.

Gemäß der Erfindung ist das Kollagenhydrolysat bevorzugt oral zu verabreichen, insbesondere in Form einer Lösung. Auf Grund seiner guten Löslichkeit kann das Kollagenhydrolysat auch verschiedenen Getränken zugesetzt werden, ohne eine Trübung zu verursachen. Durch die Verwendung von geschmacksneutralem Kollagenhydrolysat kann die Akzeptanz beim Benutzer erhöht werden.

Alternativ kann das Kollagenhydrolysat auch einem festen Lebensmittel oder Genussmittel zugesetzt werden, z.B. einem Schokoriegel (sog. Functional Food).

Günstigerweise wird das Kollagenhydrolysat in einer Menge von ca. 10 bis ca. 20 g pro Tag verabreicht, beispielsweise in einer Menge von ca. 15 g pro Tag. Diese Menge, die bei einem Körpergewicht von 75 kg etwa einem Viertel der empfohlenen täglichen Proteinzufuhr entspricht (in der Regel 0,8 g/kg Körpergewicht/Tag), ist bereits ausreichend, um einen signifikanten Effekt zu erreichen.

Sarkopenie und die mit ihr einhergehenden Symptome treten verstärkt mit fortschreitendem Alter auf. Die Verabreichung des Kollagenhydrolysats erfolgt daher insbesondere an Patienten im Alter von 50 Jahren oder mehr, bevorzugt von 60 Jahren oder mehr, weiter bevorzugt von 65 Jahren oder mehr.

Um die beschriebene Wirkung zu erreichen, wird das Kollagenhydrolysat in Verbindung mit einem Muskeltraining verabreicht.

Das Muskeltraining kann dabei jede Art von körperlicher Betätigung, bei der eine oder mehrere Muskelpartien des Körpers beansprucht werden, umfassen, besonders effektiv ist jedoch ein gezieltes Training in Form einer Wiederholung vorgegebener Übungen, insbesondere an Fitnessgeräten, wobei fast jede Muskelpartie gezielt trainiert werden kann. Es ist bekannt, dass unmittelbar nach einer derartigen Stimulation der Muskeln eine weitere Steigerung des Muskelaufbaus durch die Zufuhr entsprechender Nährstoffe induziert werden kann.

Im Rahmen der vorliegenden Erfindung ist es daher besonders günstig, wenn das Kollagenhydrolysat innerhalb von zwei Stunden, bevorzugt innerhalb von einer Stunde, an einem Patienten verabreicht wird, nachdem dieser ein Muskeltraining durchgeführt hat.

Alternativ ist es auch möglich, das Kollagenhydrolysat unmittelbar vor dem Muskeltraining zu verabreichen, zumal es dann auf Grund der guten Resorptionsfähigkeit bereits während des Trainings für den Stoffwechsel verfügbar ist. Im Gegensatz zu einigen anderen Proteinen stellt Kollagenhydrolysat auch keine besondere Belastung für den Verdauungstrakt dar.

Das Molekulargewicht des verwendeten Kollagenhydrolysats kann gemäß der Erfindung über einen sehr weiten Bereich variieren, wobei eine Obergrenze dadurch gegeben ist, dass Kollagenhydrolysat im Unterschied zu denaturiertem Kollagen oder Gelatine einen ausreichenden Hydrolysegrad aufweist, so dass es bei Raumtemperatur wasserlöslich ist und nicht geliert. Erfindungsgemäß weist das Kollagenhydrolysat ein mittleres Molekulargewicht von bis zu 5.000 Da auf, insbesondere von bis zu 3.500 Da.

Das Kollagenhydrolysat ist günstigerweise durch enzymatische Hydrolyse eines kollagenhaltigen Ausgangsmaterials hergestellt. Für diese Hydrolyse werden insbesondere Endopeptidasen und/oder Exopeptidasen mikrobiellen oder pflanzlichen Ursprungs eingesetzt.

Das kollagenhaltige Ausgangsmaterial ist in der Regel ausgewählt aus Haut oder Knochen von Wirbeltieren, bevorzugt von Säugetieren, insbesondere von Rindern oder Schweinen. Das Kollagenhydrolysat kann entweder in einem einstufigen Verfahren aus diesen Ausgangsmaterialien hergestellt sein oder über die Zwischenstufe Gelatine, wobei in diesem Fall sowohl Gelatine vom Typ A als auch vom Typ B verwendet werden kann.

Wie bereits oben erwähnt, ist die gemäß der Erfindung beobachtete Wirkung des Kollagenhydrolysats insbesondere vor dem Hintergrund bemerkenswert, dass Kollagenhydrolysat viele essentielle Aminosäuren, insbesondere Tryptophan, Leucin, Isoleucin und Valin, nicht oder nur in einem sehr geringen Anteil enthält. Es ist im Rahmen der Erfindung auch nicht notwendig, diese Aminosäuren zu supplementieren, d.h. das Kollagenhydrolysat enthält bei einer bevorzugten Ausführungsform der Erfindung keine zugesetzten freien Aminosäuren.

Gleichwohl ist eine solche Supplementierung des Kollagenhydrolysats oder eine Mischung mit anderen Komponenten im Rahmen der Erfindung möglich, um z.B. weitere ernährungsphysiologische Aspekte zu berücksichtigen. Neben freien Aminosäuren können dem Kollagenhydrolysat insbesondere (z.B. im Rahmen einer proteinreichen Diät) weitere Proteine beigemischt werden, bevorzugt Molkenprotein, Casein, Lactalbumin oder Pflanzenproteine (z.B. aus Weizen, Soja oder Erbse).

Gemäß einer weiteren Ausführungsform der Erfindung können dem Kollagenhydrolysat Vitamine und/oder Mineralstoffe zugesetzt werden, um eine ausreichende Versorgung mit den entsprechenden Stoffen sicherzustellen.

Gegenstand der vorliegenden Offenbarung ist auch ein Verfahren zur Behandlung von Sarkopenie, umfassend die wiederholte orale Verabreichung von Kollagenhydrolysat an einen Patienten.

Gemäß einem weiteren Aspekt wird durch dieses Verfahren dem degenerativen Verlust von Muskelmasse entgegengewirkt und die Muskelkraft verbessert.

Gemäß einem weiteren Aspekt wird durch das Verfahren der altersbedingte Verlust von Muskelmasse reduziert.

Gemäß einem weiteren Aspekt wird durch das Verfahren die Umwandlung von Körperfettmasse in Muskelmasse stimuliert.

Bei einer bevorzugten Ausführungsform umfasst das Verfahren ferner die Durchführung eines Muskeltrainings durch den Patienten vor mindestens einem Teil der Verabreichungen von Kollagenhydrolysat. Besonders günstig ist es, wenn die Verabreichung des Kollagenhydrolysats täglich wiederholt wird und das Muskeltraining mindestens einmal wöchentlich, bevorzugt dreimal wöchentlich.

Besonders bevorzugt ist es, wenn das Kollagenhydrolysat jeweils innerhalb von zwei Stunden, bevorzugt innerhalb von einer Stunde, nach einem Muskeltraining verabreicht wird.

Weitere Vorteile und bevorzugte Ausführungsformen des Verfahrens, insbesondere im Hinblick auf die bevorzugte Menge an Kollagenhydrolysat und die Art des Muskeltrainings, wurden bereits im Zusammenhang mit dem erfindungsgemäßen Kollagenhydrolysat als Wirkstoff beschrieben, und gelten für das Verfahren gleichermaßen.

Anhand der nachfolgend beschriebenen doppelblinden, placebokontrollierten klinischen Studie, die als Ausführungsbeispiel dient, wird die Erfindung näher erläutert.

In den Figuren zeigen
- Figur 1:: ein Säulendiagramm betreffend die fettfreie Körpermasse und die Körperfettmasse; und
- Figur 2:: ein Säulendiagramm betreffend die Muskelkraft und die sensomotorische Kontrolle.

### Beispiel

### 1. Auswahl der Studienteilnehmer

Die Studienteilnehmer wurden aus Männern im Alter von über 65 Jahren ausgewählt, bei denen nach eigenen Angaben in den letzten drei bis vier Jahren die Muskelkraft oder physische Leistungsfähigkeit deutlich zurückgegangen war. Voraussetzungen für die Teilnahme waren u.a., dass abgesehen von der Muskelschwäche keine gesundheitlichen Probleme vorlagen und die Teilnehmer in der Lage waren, ein dreimonatiges Trainingsprogramm durchzuführen.

Von 106 Personen, die nach einem Telefoninterview in die engere Auswahl genommen wurden, wurden 60 Studienteilnehmer ausgewählt, bei denen aufgrund der Messung der Kraft der Handmuskulatur mit einem Dynamometer (Trailite, LiteExpress GmbH, Coesfeld) das Vorliegen von Sarkopenie diagnostiziert werden konnte. Sarkopenie Klasse 1 wurde diagnostiziert, wenn die Handkraft mehr als eine Standardabweichung unterhalb des normalen Wertes für eine männliche Referenzpopulation im Alter von 35 bis 39 Jahren lag, Sarkopenie Klasse 2 wurde entsprechend bei einer um mehr als zwei Standardabweichungen niedrigeren Handkraft diagnostiziert.

Durch eine umfangreiche medizinische Untersuchung einschließlich eines Bluttests wurde sichergestellt, dass bei den Teilnehmern keine chronische Erkrankungen vorlagen.

Die 60 Teilnehmer der Studie wurden zufällig auf zwei Gruppen mit jeweils 30 Teilnehmern aufgeteilt, d.h. eine Behandlungsgruppe und eine Placebogruppe.

### 2. Muskeltraining

Beide Gruppen führten unter Aufsicht ein identisches Trainingsprogramm während eines Zeitraums von 12 Wochen durch, wobei dreimal pro Woche jeweils für 60 Minuten an Fitnessgeräten trainiert wurde (z.B. Kabelzuggerät, Hantelbank, Beinpresse usw.), um alle größeren Muskelpartien gezielt zu beanspruchen. Das Trainingsprogramm wurde bei jedem Teilnehmer regelmäßig überprüft und an die individuelle Leistungsfähigkeit angepasst.

Teilnehmer, die mehr als 10% der Trainingseinheiten versäumten, wurden von der Studie ausgeschlossen, so dass sich die Zahl der Teilnehmer, welche die Studie erfolgreich abschlossen und in der Auswertung berücksichtigt wurden, auf 26 in der Behandlungsgruppe auf 27 in der Placebogruppe reduzierte.

### 3. Verabreichung von Kollaqenhvdrolvsat

Die Teilnehmer der Behandlungsgruppe nahmen während der zwölfwöchigen Studiendauer täglich 15 g Kollagenhydrolysat zu sich, jeweils aufgelöst in 250 ml Wasser. Es wurde enzymatisch hydrolysiertes Kollagen aus Schweineschwarten mit einem Molekulargewicht im Bereich von 3.000 bis 3.200 Da verwendet. An den Tagen mit Muskeltraining wurden die Teilnehmer angewiesen, die Lösung so bald wie möglich, spätestens jedoch eine Stunde, nach der Trainingseinheit zu trinken.

Den Teilnehmern der Placebogruppe wurde statt Kollagenhydrolysat dieselbe Menge an Siliciumdioxid verabreicht, ebenfalls in 250 ml Wasser. Siliciumdioxid wurde verwendet, da es sich um einen sicheren Lebensmittelzusatzstoff handelt, der jedoch keinen Einfluss auf den Stoffwechsel hat.

### 4. Untersuchte Parameter

Jeweils vor und nach dem Studienzeitraum wurden die Körperfettmasse, die Knochenmasse und die fettfreie Körpermasse jedes Studienteilnehmers mittels Dual-Röntgen-Absorptiometrie (DXA) unter Verwendung eines Strators DR 2D Fan Beam (Degen Medizintechnik, Heppenheim) gemessen. Sofern das Körpergewicht jeweils gleich bleibt, kann aus einer Verringerung des Fettanteils bzw. einer Erhöhung des fettfreien Anteils auf eine Zunahme an Muskelmasse geschlossen werden.

Als Parameter für die Muskelkraft wurde vor und nach dem Studienzeitraum die isokinetische Kraft des Quadrizeps des rechten Beins gemessen (Con-Trex, Dübendorf, Schweiz).

Die sensomotorische Kontrolle vor und nach dem Studienzeitraum wurde mittels eines standardisierten einbeinigen Stabilisationstests bestimmt (Posturomed, Haider-Bioswing, Weiden). Bei diesem Test ist der Messwert umso niedriger, je besser die sensomotorische Kontrolle des Probanden ist, was unter anderem mit der Muskelkraft korreliert.

### 5. Ergebnisse

Das durchschnittliche Gewicht der Studienteilnehmer blieb während des Studienzeitraums im Wesentlichen gleich (85,6 kg vor der Studie und 85,0 kg nach der Studie), wobei es auch innerhalb der beiden Gruppen keine statistisch signifikante Gewichtsänderung durch die Studie gab.

Sowohl bei einer Gesamtbetrachtung aller Teilnehmer (n=53) als auch innerhalb beider Gruppen (Behandlungsgruppe, n=26 und Placebogruppe, n=27) ergab sich jedoch eine deutliche, d.h. statistisch signifikante Zunahme der fettfreien Körpermasse, der Knochenmasse und der Muskelkraft, sowie eine Abnahme der Fettmasse und der sensomotorischen Kontrolle. Dieses Ergebnis ist allein aufgrund des von allen Studienteilnehmern durchgeführten Krafttrainings zu erwarten.

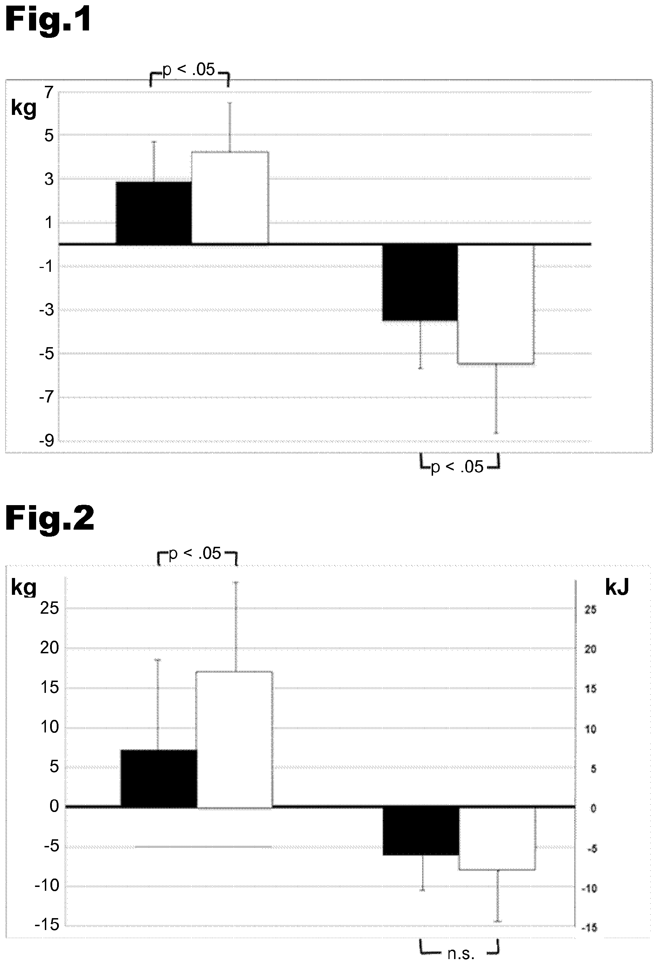

Der Beleg für die Wirksamkeit des Kollagenhydrolysats im Sinne der vorliegenden Erfindung ergibt sich aus einem Vergleich der gemessenen Parameter zwischen der Behandlungsgruppe und der Placebogruppe. In der Figur 1 sind in Form eines Säulendiagramms die durchschnittliche Zunahme der fettfreien Körpermasse (linker Bereich) und die Abnahme der Körperfettmasse (rechter Bereich) in kg dargestellt, jeweils für die Placebogruppe als schwarzer Balken und für die Behandlungsgruppe als weißer Balken. In der Figur 2 ist die durchschnittliche Zunahme der Muskelkraft in kg (linker Bereich) und die durchschnittliche Abnahme der sensomotorischen Kontrolle in kJ (rechter Bereich) dargestellt, ebenfalls als schwarze Balken für die Placebogruppe und als weiße Balken für die Behandlungsgruppe.

Sowohl die Zunahme der fettfreien Körpermasse, die Abnahme der Körperfettmasse und die Zunahme der Muskelkraft sind jeweils bei der Behandlungsgruppe signifikant stärker ausgeprägt als bei der Placebogruppe (p<0,05). Auch die Verbesserung der sensomotorischen Kontrolle ist bei der Behandlungsgruppe stärker, allerdings nicht in statistisch signifikantem Ausmaß.

Die Studie zeigt somit eindeutig, dass durch die Verabreichung von Kollagenhydrolysat einem degenerativem Verlust von Muskelmasse entgegengewirkt und eine Verbesserung der Muskelkraft erzielt werden kann, und dass Kollagenhydrolysat als Wirkstoff zur Behandlung von Sarkopenie geeignet ist.

## Patentansprüche

1. Kollagenhydrolysat als Wirkstoff zur Behandlung von Sarkopenie oder von degenerativem Verlust von Muskelmasse, wobei das Kollagenhydrolysat ein mittleres Molekulargewicht von bis zu 5.000 Da aufweist und keine zugesetzten freien Aminosäuren enthält, und wobei das Kollagenhydrolysat in Verbindung mit einem Muskeltraining zu verabreichen ist.

2. Nicht-therapeutische Verwendung von Kollagenhydrolysat als Wirkstoff zur Verbesserung der Muskelkraft, wobei das Kollagenhydrolysat ein mittleres Molekulargewicht von bis zu 5.000 Da aufweist und keine zugesetzten freien Aminosäuren enthält, und wobei das Kollagenhydrolysat in Verbindung mit einem Muskeltraining zu verabreichen ist.

3. Nicht-therapeutische Verwendung von Kollagenhydrolysat als Wirkstoff zur Reduktion des altersbedingten Verlustes von Muskelmasse, wobei das Kollagenhydrolysat ein mittleres Molekulargewicht von bis zu 5.000 Da aufweist und keine zugesetzten freien Aminosäuren enthält, und wobei das Kollagenhydrolysat in Verbindung mit einem Muskeltraining zu verabreichen ist.

4. Nicht-therapeutische Verwendung von Kollagenhydrolysat als Wirkstoff zur Stimulation der Umwandlung von Körperfettmasse in Muskelmasse, wobei das Kollagenhydrolysat ein mittleres Molekulargewicht von bis zu 5.000 Da aufweist, und wobei das Kollagenhydrolysat in Verbindung mit einem Muskeltraining zu verabreichen ist .

5. Kollagenhydrolysat zur Behandlung oder die Verwendung nach einem der vorangehenden Ansprüche, wobei das Kollagenhydrolysat oral zu verabreichen ist, bevorzugt in Form einer Lösung.

6. Kollagenhydrolysat zur Behandlung oder die Verwendung nach Anspruch 5, wobei das Kollagenhydrolysat in einer Menge von 10 bis 20 g pro Tag zu verabreichen ist, bevorzugt in einer Menge von 15 g pro Tag.

7. Kollagenhydrolysat zur Behandlung oder die Verwendung nach einem der vorangehenden Ansprüche, wobei das Kollagenhydrolysat an einen Patienten im Alter von 50 Jahren oder mehr zu verabreichen ist, bevorzugt von 60 Jahren oder mehr, weiter bevorzugt von 65 Jahren oder mehr.

8. Kollagenhydrolysat zur Behandlung oder die Verwendung nach einem der vorangehenden Ansprüche, wobei das Kollagenhydrolysat innerhalb von zwei Stunden, bevorzugt innerhalb von einer Stunde, an einen Patienten zu verabreichen ist, nachdem dieser ein Muskeltraining durchgeführt hat, oder wobei das Kollagenhydrolysat unmittelbar vor einem Muskeltraining an einen Patienten zu verabreichen ist.

9. Kollagenhydrolysat zur Behandlung oder die Verwendung nach einem der vorangehenden Ansprüche, wobei das Kollagenhydrolysat ein mittleres Molekulargewicht von bis zu 3.500 Da aufweist.

10. Kollagenhydrolysat zur Behandlung oder die Verwendung nach einem der vorangehenden Ansprüche, wobei das Kollagenhydrolysat durch enzymatische Hydrolyse eines kollagenhaltigen Ausgangsmaterials hergestellt ist, welches bevorzugt ausgewählt ist aus Haut oder Knochen von Wirbeltieren, weiter bevorzugt von Säugetieren, insbesondere von Rindern oder Schweinen.

## Claims

1. Collagen hydrolysate as an active substance for treatment of sarcopenia or degenerative loss of muscle mass, wherein the collagen hydrolysate has a mean molecular weight of up to 5.000 Da and does not contain any added free amino acids, and wherein the collagen hydrolysate is to be administered in conjunction with muscle exercise.

2. Non-therapeutic use of collagen hydrolysate as an active substance for improving muscle strength, wherein the collagen hydrolysate has a mean molecular weight of up to 5.000 Da and does not contain any added free amino acids, and wherein the collagen hydrolysate is to be administered in conjunction with muscle exercise.

3. Non-therapeutic use of collagen hydrolysate as an active substance for the reduction of the age-related loss of muscle mass, wherein the collagen hydrolysate has a mean molecular weight of up to 5.000 Da and does not contain any added free amino acids, and wherein the collagen hydrolysate is to be administered in conjunction with muscle exercise.

4. Non-therapeutic use of collagen hydrolysate as an active substance for the stimulation of the conversion of body fat mass into muscle mass, wherein the collagen hydrolysate has a mean molecular weight of up to 5.000 Da, and wherein the collagen hydrolysate is to be administered in conjunction with muscle exercise.

5. Collagen hydrolysate for treatment or the use according to one of the preceding claims, wherein the collagen hydrolysate is to be administered orally, preferably in the form of a solution.

6. Collagen hydrolysate for treatment or the use according to claim 5,
wherein the collagen hydrolysate is to be administered in a quantity of 10 to 20 g per day, preferably in a quantity of 15 g per day.

7. Collagen hydrolysate for treatment or the use according to one of the preceding claims, wherein the collagen hydrolysate is to be administered to a patient aged 50 years or more, preferably 60 years or more, more preferably 65 years or more.

8. Collagen hydrolysate for treatment or the use according to one of the preceding claims, wherein the collagen hydrolysate is to be administered to a patient within two hours, preferably within one hour, after said patient has performed a muscle exercise, or wherein the collagen hydrolysate is to be administered to a patient immediately before a muscle exercise.

9. Collagen hydrolysate for treatment or the use according to one of the preceding claims, wherein the collagen hydrolysate has a mean molecular weight of up to 3,500 Da.

10. Collagen hydrolysate for treatment or the use according to one of the preceding claims, wherein the collagen hydrolysate is manufactured by the enzymatic hydrolysis of a collagen-containing starting material, which is preferably selected from skin or bone of vertebrates, more preferably from mammals, particularly from cattle or pigs.

## Revendications

1. Hydrolysat de collagène comme principe actif pour le traitement de la sarcopénie ou de la perte dégénérative de masse musculaire, dans lequel l'hydrolysat de collagène présente une masse moléculaire moyenne allant jusqu'à 5 000 Da et ne contient pas d'acides aminés libres ajoutés, et dans lequel l'hydrolysat de collagène est à administrer en association avec un entraînement musculaire.

2. Utilisation non thérapeutique d'hydrolysat de collagène comme principe actif pour l'amélioration de la force musculaire, dans laquelle l'hydrolysat de collagène présente une masse moléculaire moyenne allant jusqu'à 5 000 Da et ne contient pas d'acides aminés libres ajoutés, et dans laquelle l'hydrolysat de collagène est à administrer en association avec un entraînement musculaire.

3. Utilisation non thérapeutique d'hydrolysat de collagène comme principe actif pour la réduction de la perte de masse musculaire liée à l'âge, dans laquelle l'hydrolysat de collagène présente une masse moléculaire moyenne allant jusqu'à 5 000 Da et ne contient pas d'acides aminés libres ajoutés, et dans laquelle l'hydrolysat de collagène est à administrer en association avec un entraînement musculaire.

4. Utilisation non thérapeutique d'hydrolysat de collagène comme principe actif pour la stimulation de la transformation de masse corporelle adipeuse en masse musculaire, dans laquelle l'hydrolysat de collagène présente une masse moléculaire moyenne allant jusqu'à 5 000 Da, et dans laquelle l'hydrolysat de collagène est à administrer en association avec un entraînement musculaire.

5. Hydrolysat de collagène pour le traitement ou l'utilisation selon l'une quelconque des revendications précédentes, dans lequel l'hydrolysat de collagène est à administrer par voie orale, de préférence sous forme d'une solution.

6. Hydrolysat de collagène pour le traitement ou l'utilisation selon la revendication 5, dans lequel l'hydrolysat de collagène est à administrer dans une quantité de 10 à 20 g par jour, de préférence dans une quantité de 15 g par jour.

7. Hydrolysat de collagène pour le traitement ou l'utilisation selon l'une quelconque des revendications précédentes, dans lequel l'hydrolysat de collagène est à administrer à un patient âgé de 50 ans ou plus, de préférence de 60 ans ou plus, plus préférablement de 65 ans ou plus.

8. Hydrolysat de collagène pour le traitement ou l'utilisation selon l'une quelconque des revendications précédentes, dans lequel l'hydrolysat de collagène est à administrer à un patient deux heures, de préférence une heure après que celui-ci a effectué un entraînement musculaire, ou dans lequel l'hydrolysat de collagène est à administrer à un patient directement avant un entraînement musculaire.

9. Hydrolysat de collagène pour le traitement ou l'utilisation selon l'une quelconque des revendications précédentes, dans lequel l'hydrolysat de collagène présente une masse moléculaire moyenne allant jusqu'à 3 500 Da.

10. Hydrolysat de collagène pour le traitement ou l'utilisation selon l'une quelconque des revendications précédentes, dans lequel l'hydrolysat de collagène est produit par hydrolyse enzymatique d'un matériau de départ contenant du collagène, lequel est sélectionné de préférence à partir de peau ou d'os de vertébrés, plus préférablement de mammifères, en particulier de bovins ou de cochons.
